# EUROPÄISCHE PATENTSCHRIFT

(11) **EP 1 741 455 B1**
(45) Veröffentlichungstag und Bekanntmachung des Hinweises auf die Patenterteilung: **02.11.2011**
(21) Anmeldenummer: 06010459.3
(22) Anmeldetag: 20.05.2006
(51) Int. Cl.: A61L 24/06, A61L 27/16, A61L 27/50

(54) **Gefärbter Polymethylmethacrylat-Knochenzement und Verfahren zu seiner Herstellung**
Coloured polymethylmethacrylate bone cement and method for preparing the same
Ciment osseux coloré à base de polyméthyl méthacrylate et son procédé de préparation

(30) Priorität: 07.07.2005 DE 102005032110
(43) Veröffentlichungstag der Anmeldung: 10.01.2007
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(56) Entgegenhaltungen:
- US-A- 4 588 583
- LEE C L ET AL: "LASER ABLATION OF DYED ACRYLIC BONE CEMENT" LASERS IN SURGERY AND MEDICINE, WILEY- LISS, NEW YORK, US, Bd. 20, Nr. 3, 1. Januar 1997 (1997-01-01), Seiten 280-289, XP000694435 ISSN: 0196-8092
- BARGAR W L; MARTIN R B; DEJESUS R; MADISON M T: "The addition of tobramycin to contrast bone cement. Effect on flexural strength." THE JOURNAL OF ARTHROPLASTY, Bd. 1, Nr. 3, 1986, Seiten 165-168, XP002557137 USA

## Beschreibung

Der Gegenstand der Erfindung ist ein gefärbter Polymethylmethacrylat-Knochenzement.

Polymethylmethacrylat-Knochenzemente (PMMA-Knochenzemente) finden seit Jahrzehnten breite Anwendung in der Medizin zur Verankerung von Endoprothesen im Knochen (Klaus-Dieter Kühn: Knochenzemente für die Endoprothetik: ein aktueller Vergleich der physikalischen und chemischen Eigenschaften handelsüblicher PMMA-Zemente. Springer Verlag Berlin Heidelberg New York, 2001). Polymethylmethacrylat-Knochenzemente sind im Allgemeinen aus einer flüssigen Monomerkomponente und einer Pulverkomponente aufgebaut. Die flüssige Monomerkomponente besteht aus Methylmethacrylat und einem Aktivator. Als Aktivator wird N,N-Dimethyl-p-toluidin bevorzugt verwendet. Die Pulverkomponente besteht in der Regel aus Polymethylmethacrylat oder Polymethylmethacrylat-co-methylacrylat, einem Röntgenkontrastmittel und einem radikalischen Initiator. Als Röntgenkontrastmittel sind Zirkoniumdioxid und Bariumsulfat üblich. Dibenzoylperoxid wird als radikalischer Initiator bevorzugt eingesetzt. Nach Vermischen der Monomerkomponente und der Pulverkomponente härtet der Knochenzement durch radikalische Polymerisation des Monomers innerhalb weniger Minuten aus.

Übliche Polymethylmethacrylat-Knochenzemente liegen nach dem Anmischen als weiße bis schwach gelbliche pastöse Massen vor. Dadurch ist mitunter eine optische Differenzierung zwischen dem Knochenzement und dem Knochengewebe bei der Einbringung des angemischten Knochenzementes in den Knochen problematisch. Es ist jedoch wünschenswert, dass der Knochenzement visuell problemlos vom umliegenden Knochengewebe unterschieden werden kann.

Aus diesem Grund haben seit ungefähr 30 Jahren die von der Firma Heraeus Kulzer GmbH hergestellten Polymethylmethacrylat-Knochenzemente eine grüne Farbe. Diese Farbe wird durch eine grüne Monomerkomponente und eine grüne Pulverkomponente erzielt. In beiden Komponenten ist als Farbstoff Chlorophyllin enthalten.

Das Chlorophyllin ist bei den Polymethylmethacrylat-Knochenzementen der Firma Heraeus Kulzer GmbH mit Hilfe von raffiniertem Erdnussöl (Biskin®) als Löslichkeitsvermittler, in der flüssigen Monomerkomponente gelöst. Neben der gefärbten Monomerkomponente können Polymethylmethacrylat-Knochenzemente auch eine gefärbte Pulverkomponente enthalten. Eine an sich bekannte Methode zur Färbung der Pulverkomponente der Polymethylmethacrylat-Knochenzemente besteht darin, gefärbte Polymethylmethacrylat- oder Polymethylmethacrylat-co-methylacrylat-Partikel zu verwenden. Diese können zur Eigenschaftsbeeinflussung der Polymethylmethacrylat-Knochenzemente mit einem nichtgefärbten zweiten Polymer kombiniert werden. Ein dabei auftretendes Problem besteht darin, die Farbe, den Farbeindruck, der Pulverkomponente auch bei unterschiedlichen Mischungsverhältnissen des gefärbten Polymers zum nichtgefärbten Polymers sicher zu reproduzieren.

In dem Artikel von C. Lee et al., Lasers in Surgery and Medicine 20, 280-289 (1997), wird ein Polymethylmethacrylat-Knochenzement mit einem blauen oder roten Farbstoff in der Pulverkomponente beschrieben.

Die Synthese von gefärbten Polymethylmethacrylat- oder Polymethylmethacrylat-co-methylacrylat-Partikeln, bei welcher der Farbstoff während der Perlpolymerisation in die entstehenden Polymerperlen eingeschlossen wird, ist unter technischen Bedingungen sehr kompliziert und aufwendig. Ein wesentlicher Grund dafür ist die mitunter geringe Beständigkeit von Farbstoffen gegenüber den bei Perlpolymerisationen eingesetzten radikalischen Initiatoren und gegenüber den bei der Polymerisation auftretenden Radikalen. Insbesondere die Initiatoren können Oxidationsprozesse verursachen und damit den Farbstoff entfärben.

Die konstante farbliche Beschaffenheit der Polymethylmethacrylat-Knochenzemente ist für die Akzeptanz der Knochenzemente beim Kunden ein wesentlicher Faktor und damit von wirtschaftlicher Bedeutung.

Der Erfindung liegt deshalb die Aufgabe zu Grunde, einen gefärbten Polymethylmethacrylat-Knochenzement zu entwickeln, der die bekannten Probleme der bisher üblichen Polymethylmethacrylat-Knochenzemente überwindet. Die Pulverkomponente des Polymethylmethacrylat-Knochenzementes soll so beschaffen sein, dass die Farbe sicher reproduziert werden kann. Es soll möglich sein, kostengünstige ungefärbte Polymere, wie Polymethylmethacrylat oder Polymethylmethacrylat-co-methylacrylat oder Polymethylmethacrylat-co-styren sowie ähnlich aufgebaute Copolymere oder Terpolymere zur Herstellung von gefärbten Polymethylmethacrylat-Knochenzemente einsetzen zu können. Wichtig ist dabei, dass der Farbeindruck des Knochenzementes in der Pulverkomponente gleichmäßig ist. Das bedeutet, die Farbe der Pulverkomponente muss visuell erkennbar homogen sein. Weiterhin ist es wichtig, dass die Pulverkomponente des Polymethylmethacrylat-Knochenzementes in ihrer Rieselfähigkeit sich nicht von der Rieselfähigkeit und dem Anquellverhalten von ungefärbten Polymethylmethacrylat-Knochenzementen unterscheidet.

Die Aufgabe wurde erfindungsgemäß durch einen gefärbten Polymethylmethacrylat-Knochenzement gelöst, bei dem mindestens die Oberfläche der Polymerpartikel der Pulverkomponente mit einem Gemisch aus einem oder mehreren Farbstoffen und einem hydrophoben, niedermolekularen oder oligomeren, organischen Haftvermittler teilweise oder vollständig beschichtet ist, wobei eine solche Menge an Haftvermittler vorhanden ist, dass die Polymerpartikel visuell erkennbar nicht angequollen sind. Durch die Verwendung eines hydrophoben Haftvermittlers ist es möglich, gleichmäßig geringe Farbstoffmengen auf die Polymerpartikel so aufzubringen, dass diese fest auf den Polymerpartikeln haften. Dadurch wird der Farbstoff oder die Farbstoffe auf der Partikeloberfläche fixiert. Wichtig ist dabei, dass nur geringe Mengen an Haftvermittler vorhanden sind. Größere Mengen Haftvermittler könnten die Polymerpartikel anquellen oder anlösen und diese dadurch verkleben. Das Rieselverhalten und damit auch die Quellfähigkeit der Pulverkomponente würde sich dadurch gegenüber einem ungefärbten Polymethylmethacrylat-Knochenzement deutlich verändern.

Gegebenenfalls kann auch die Oberfläche der Röntgenkontrastmittel Bariumsulfat und/oder Zirkoniumoxid oder gegebenenfalls die Oberfläche aller Bestandteile der Pulverkomponente mit einem Gemisch aus einem oder mehreren Farbstoffen und einem hydrophoben, niedermolekularen oder oligomeren, organischen Haftvermittler teilweise oder vollständig beschichtet sein. Bevorzugt sind der oder die Farbstoffe im hydrophoben, niedermolekularen oder oligomeren, organischen Haftvermittler löslich oder in diesem suspendiert. So löst sich zum Beispiel Chlorophyllin (E141) in Biskin oder in Ölsäureethylester. Unter dem Begriff "suspendiert" wird verstanden, dass die Farbstoffpartikel Korngrößen kleiner gleich 1 µm haben und im Haftvermittler homogen verteilt sind.

Weiterhin sind Ölsäureester und/oder Elaidinsäureester und/oder Linolsäureester und/oder Linolensäureester der aliphatischen Alkohole mit 1 bis 22 Kohlenstoffatomen oder Oligomere dieser Ester als Haftvermittler bevorzugt.

Ferner sind, Methacrylsäureester oder Acrylsäureester der aliphatischen Alkohole mit 4 bis 16 Kohlenstoffatomen oder Oligomere dieser Methacrylsäureester oder Acrylsäureester mit einer Molmasse kleiner 3.000 g/mol als Haftvermittler bevorzugt. Es ist auch möglich, Oligomere dieser Struktur mit Molmassen größer 3.000 g/mol als Haftvermittler zu verwenden, sofern diese bei Raumtemperatur pastös oder zähflüssig sind.

Besonders sind Ölsäure, Elaidinsäure, Linolensäure, Glycerintrioleat, Glycerinelaidinat, Glycerintrilinolenat, Ethylenglykoldioleinat, Ethylenglykoldielaidinat, Ethylenglykoltrilinolenat und deren Oligomere als Haftvermittler bevorzugt. Diese Oligomere können durch Einwirkung von Luft bei erhöhter Temperatur als sogenannte Blasöle oder auch durch Erwärmung in Abwesenheit von Luftsauerstoff als sogenannte Standöle hergestellt sein. Es können auch Mischester des Glycerins, des Ethylenglykols, des Sorbitols, des Mannitols, des Xylitols, des Erythrols, des 1,1,1-Trimethylolpropans mit den ungesättigten Fettsäuren Ölsäure, Elaidinsäure, Linolensäure und der Arachidonsäure sowie den davon abgeleiteten Oligomeren als Haftvermittler eingesetzt werden.

Bevorzugt sind in einer Ausführungsform Haftvermittler, die synthetisch oder partiell synthetisch hergestellt sind und die keine Proteine oder Abbauprodukte von Proteinen enthalten. Dieses Charakteristikum ist besonders wichtig, weil bei der Verwendung von proteinfreien Haftvermittlern das Risiko des Auftretens von Allergien minimiert ist.

Es könne aber auch raffiniertes Erdnussöl, gehärtetes Leinöl, gehärtetes Rapsöl und Sonnenblumenöl als Haftvermittler eingesetzt. Auch möglich ist der Einsatz von weiteren, in der menschlichen Ernährung üblichen Fetten und pflanzlichen Ölen.

Der Haftvermittler sollte zweckmäßigerweise radikalisch polymerisierbare Doppelbindungen enthalten. Dadurch kann der Haftvermittler beim Aushärten des Knochenzementes an der Polymerisation mit teilnehmen und wird fest im Knochenzement integriert.

Außerdem ist es zweckmäßig, dass Gemische aus dem Haftvermittler und dem Farbstoff oder den Farbstoffen in Methylmethacrylat oder auch Gemischen von Methylmethacrylat mit anderen Methacrylsäureestern, wie Methacrylsäureethylester, Methacrylsäureisobornylester und Methacrylsäure-2-ethyl-hexylester, und Acrylsäureestern, wie Acrylsäuremethylester, löslich sind. Bei dem Vermischen der Pulverkomponente mit der flüssigen Monomerkomponente kann sich das Gemisch aus dem Farbstoff oder den Farbstoffen und dem Haftvermittler in der flüssigen Monomerkomponente lösen und auch diese färben. Das bedeutet, die Beschichtung wird zumindestens teilweise durch Einwirkung des Monomers abgelöst und bildet mit dem Monomer eine Lösung. Dadurch wird ein gleichmäßiger Farbeindruck des Polymethylmethacrylat-Knochenzementes bei der Aushärtung erreicht, weil auch das radikalisch polymerisierende Monomer den Farbstoff gelöst enthält und damit während der Aushärtung zu einem farbig erscheinenden Polymer polymerisiert.

Besonders bevorzugt als Farbstoffe sind Chlorophyll, Chlorophyllin (E141), Indigo, Malachitgrün, Kristallviolett, Brilliantblau, Brilliantgrün, Kupferphthalocyanin, Kobaltphthalocyanin, Carotin, Vitamin B12 und davon abgeleitete Derivate.

Ein erfindungsgemäßes Verfahren zur Färbung der geschilderten Pulverkomponente besteht im wesentlichen darin, dass die Polymerpartikel oder Gemische aus den Polymerpartikeln und dem Röntgenkontrastmittel oder Gemische aus den Polymerpartikeln, dem Röntgenkontrastmittel und dem Initiator mit einer flüssigen oder pastösen Mischung des Farbstoffes oder der Farbstoffe und dem Haftvermittler durch Vermischen im Temperaturbereich von 0 °C bis 50 °C in Gegenwart von Luft oder Inertgas so beschichtet werden, dass die Schichtdicke des Gemisches auf den beschichteten Partikeln kleiner 2 µm ist und das die beschichteten Partikel nicht miteinander verklebt sind. Es ist dabei besonders vorteilhaft, wenn der Mischvorgang so ausgeführt wird, dass der Glaspunkt der Polymerpartikel nicht überschritten wird. Ein Überschreiten des Glaspunktes führt zum Verkleben der Polymerpartikel und damit zur Bildung von Agglomeraten. Der Mischvorgang kann mit Vorteil in technisch üblichen Mischern, wie Rührwerksmischern oder Röhnradmischern ausgeführt werden. Vorteilhaft lässt sich die Beschichtung bei Temperaturen um 40 °C ausführen, weil die Viskosität der erwärmten Haftvermittler niedriger ist als die Viskosität der Haftvermittler bei Raumtemperatur. Dadurch ist eine gleichmäßige Verteilung der Haftvermittler leichter möglich.

Die Erfindung soll durch nachstehende Beispiele erläutert werden, ohne jedoch die Erfindung zu beschränken.

### Beispiel 1:

Es werden in einer Plastikflasche mit Schraubverschluss 33,2 g eines Poly-methylmethacrylatco-methylacrylates (Molmasse ~ 600.000 g/mol, Partikelgröße 4-50 µm) mit 4,0 mg eines Gemischs, bei dem 1,0 mg Chlorophyll in 3,0 mg Ölsäureethylester gelöst sind, in einem Turbula-Taumelmischer 24 Stunden bei Raumtemperatur gemischt. Nach 24 Stunden hat das zuvor farblose Polymer eine grünliche Färbung angenommen. Die Polymerpartikel sind nicht angequollen und auch nicht miteinander verklebt. Anschließend werden 6,3 g Zirkoniumdioxid und 0,84 g Dibenzoylperoxid (phlegmatisiert mit 25 % Wasser) zu dem gefärbten Polymer gegeben und es wird 10 Minuten bei Raumtemperatur mit Hilfe des Turbula-Taumelmischers gemischt. Das entstandene, visuell homogen erscheinende, rieselfähige Gemisch wird als Pulverkomponente eines Polymethylmethacrylat-Knochenzementes verwendet.

### Beispiel 2:

Es werden in einer Plastikflasche mit Schraubverschluss 33,2 g eines Poly-methylmethacrylatco-methylacrylates (Molmasse ~ 600.000 g/mol, Partikelgröße 5-40 µm) und 6,3 g Zirkoniumdioxid mit 3,0 mg eines Gemischs, bei dem 1,0 mg Chlorophyll in 2,0 mg Ölsäureethylester gelöst sind, in einem Turbula-Taumelmischer 24 Stunden bei Raumtemperatur gemischt. Nach 24 Stunden hat das zuvor farblose Polymer eine grünliche Färbung angenommen. Anschließend werden 0,84 g Dibenzoylperoxid (phlegmatisiert mit 25 % Wasser) zu dem gefärbten Gemisch gegeben und 10 Minuten wird bei Raumtemperatur mit Hilfe des Turbula-Taumelmischers gemischt. Das entstandene, visuell homogen erscheinende Gemisch wird als Pulverkomponente eines Polymethylmethacrylat-Knochenzementes verwendet.

### Beispiel 3:

Es wird in analoger Weise wie bei Beispiel 1 die Pulverkomponente eines Polymethylmethacrylat-Kochenzementes hergestellt, wobei jedoch 4,0 mg eines Gemischs, das aus 1,0 mg Chlorophyll und 3,0 mg Glycerintrioleinat besteht, verwendet wird.

### Beispiel 4:

Es wird in analoger Weise wie bei Beispiel 1 die Pulverkomponente eines Polymethylmethacrylat-Kochenzementes hergestellt, wobei jedoch 4,0 mg eines Gemischs, das aus 1,0 mg Brilliantblau und 3,0 mg Ölsäure besteht, verwendet wird.

### Beispiel 5:

Eine flüssige Monomerkomponente wird aus 18,40 g Methylmethacrylat und 0,38 g N,N-Dimethyl-p-toluidin durch Vermischen hergestellt. Dieses Gemisch stellt die Monomerkomponente der folgenden Zemente dar.

Es werden jeweils 39,00 g der Pulverkomponente der Beispiele 1-4 mit jeweils 18,00 g der Monomerkomponente kombiniert. Bei Vermischen der Pulverkomponente mit der flüssigen Monomerkomponente entsteht bei Raumtemperatur nach 1 Minute ein grüner, plastisch verformbarer Teig. Dieser ist 3 Minuten verarbeitbar und härtet danach aus. Es bildet sich ein grüner Festkörper.

### Beispiel 6:

Eine flüssige Monomerkomponente wird aus 18,40 g Methylmethacrylat, 0,38 g N,N-Dimethyl-p-toluidin und 1,0 mg Chlorophyll, das in 2,0 mg Ölsäureethylester gelöst ist, durch Vermischen hergestellt. Dieses grüne Gemisch stellt die Monomerkomponente der folgenden Zemente dar.

Es werden jeweils 39,00 g der Pulverkomponente der Beispiele 1-4 mit jeweils 18,00 g der Monomerkomponente kombiniert. Bei Vermischen der Pulverkomponente mit der flüssigen Monomerkomponente entsteht bei Raumtemperatur nach 1 Minute ein grüner, plastisch verformbarer Teig. Dieser ist 3 Minuten verarbeitbar und härtet danach zu einem grünen Festkörper aus.

## Patentansprüche

1. Gefärbter Polymethylmethacrylat-Knochenzement aus einer flüssigen und einer Pulverkomponente, **dadurch gekennzeichnet, dass** mindestens die Oberfläche der Polymerpartikel der Pulverkomponente mit einem Gemisch aus einem oder mehreren Farbstoffen und einem hydrophoben, niedermolekularen oder oligomeren, organischen Haftvermittler teilweise oder vollständig beschichtet ist, wobei eine solche Menge an Haftvermittler vorhanden ist, dass die Polymerpartikel visuell erkennbar nicht angequollen sind.

2. Gefärbter Polymethylmethacrylat-Knochenzement nach Anspruch 1 enthaltend zusätzlich in der Pulverkomponente Bariumsulfat und/oder Zirkoniumoxid, **dadurch gekennzeichnet, dass** auch die Oberfläche der Röntgenkontrastmittel Bariumsulfat und/oder Zirkoniumoxid oder die Oberfläche aller Bestandteile der Pulverkomponente mit einem Gemisch aus einem oder mehreren Farbstoffen und einem hydrophoben, niedermolekularen oder oligomeren, organischen Haftvermittler teilweise oder vollständig beschichtet ist.

3. Gefärbter Polymethylmethacrylat-Knochenzement nach einem der Ansprüche 1 und 2, **dadurch gekennzeichnet, dass** der oder die Farbstoffe im hydrophoben, niedermolekularen oder oligomeren, organischen Haftvermittler gelöst oder in diesem suspendiert sind.

4. Gefärbter Polymethylmethacrylat-Knochenzement nach einem der Ansprüche 1-3, **dadurch gekennzeichnet, dass** das Masseverhältnis Farbstoff/Farbstoffe zu hydrophobem, niedermolekularen oder oligomeren, organischen Haftvermittler 1,0 zu 0,1 bis 1,0 zu 10,0 ist.

5. Gefärbter Polymethylmethacrylat-Knochenzement nach einem der Ansprüche 1-4, **dadurch gekennzeichnet, dass** Ölsäureester und/oder Elaidinsäureester und/oder Linolsäureester und/oder Linolensäureester der aliphatischen Alkohole mit 1 bis 22 Kohlenstoffatomen oder Oligomere dieser Ester als Haftvermittler vorhanden sind.

6. Gefärbter Polymethylmethacrylat-Knochenzement nach einem der Ansprüche 1-5, **dadurch gekennzeichnet, dass** Methacrylsäureester oder Acrylsäureester der aliphatischen Alkohole mit 4 bis 16 Kohlenstoffatomen oder Oligomere dieser Methacrylsäureester oder Acrylsäureester mit einer Molmasse kleiner 3.000 g/mol als Haftvermittler vorhanden sind.

7. Gefärbter Polymethylmethacrylat-Knochenzement nach einem der Ansprüche 1-6, **dadurch gekennzeichnet, dass** Ölsäure, Elaidinsäure, Linolensäure, Glycerintrioleat, Glycerinelaidinat, Glycerintrilinolenat, Ethylenglykoldioleinat, Ethylenglykoldielaidinat, Ethylenglykoltrilinolenat und deren Oligomere als Haftvermittler vorhanden sind.

8. Gefärbter Polymethylmethacrylat-Knochenzement nach einem der Ansprüche 1-7, **dadurch gekennzeichnet, dass** Haftvermittler vorhanden sind, die synthetisch oder partiell synthetisch hergestellt sind und die keine Proteine oder Abbauprodukte von Proteinen enthalten.

9. Gefärbter Polymethylmethacrylat-Knochenzement nach einem der Ansprüche 1-7, **dadurch gekennzeichnet, dass** als Haftvermittler raffiniertes Erdnussöl, gehärtetes Leinöl, gehärtetes Rapsöl und Sonnenblumenöl vorhanden sind.

10. Gefärbter Polymethylmethacrylat-Knochenzement nach einem der Ansprüche 1-9, **dadurch gekennzeichnet, dass** der Haftvermittler radikalisch polymerisierbare Doppelbindungen enthält.

11. Gefärbter Polymethylmethacrylat-Knochenzement nach einem der Ansprüche 1-10, **dadurch gekennzeichnet, dass** Gemische aus dem Haftvermittler und dem Farbstoff oder den Farbstoffen in Methylmethacrylat löslich sind.

12. Gefärbter Polymethylmethacrylat-Knochenzement nach einem der Ansprüche 1-11, **dadurch gekennzeichnet, dass** als Farbstoffe Chlorophyll, Chlorophyllin, Indigo, Malachitgrün, Kristallviolett, Brilliantblau, Brilliantgrün, Kupferphthalocyanin, Kobaltphthalocyanin, Carotin, Vitamin B12 und davon abgeleitete Derivate als Farbstoffe vorhanden sind.

13. Verfahren zur Färbung der Polymerpartikel der Pulverkomponente von PMMA Knochenzement, **dadurch gekennzeichnet, dass** die Polymerpartikel oder Gemische aus den Polymerpartikeln und dem Röntgenkontrastmittel oder Gemische aus den Polymerpartikeln, dem Röntgenkontrastmittel und dem Initiator mit einer flüssigen oder pastösen Mischung des Farbstoffes oder der Farbstoffe und dem Haftvermittler durch Vermischen im Temperaturbereich von 0 °C bis 50 °C in Gegenwart von Luft oder Inertgas so beschichtet werden, dass die Schichtdicke des Gemisches auf den beschichteten Partikeln kleiner 2 µm ist und dass die beschichteten Partikel nicht miteinander verklebt sind.

## Claims

1. Dyed polymethylmethacrylate bone cement made of a liquid and a powder component, **characterised in that** at least the surface of the polymer particles of the powder component is partly or fully coated with a mixture of one or more dyes and a hydrophobic low-molecular or oligomeric organic bonding agent, whereby the quantity of the bonding agent present is such that the polymer particles are not swollen to a visibly detectable degree.

2. Dyed polymethylmethacrylate bone cement according to claim 1, additionally containing barium sulfate and/or zirconium oxide in the powder component, **characterised in that** the surface of the X-ray contrast medium, barium sulfate and/or zirconium oxide, also or the surface of all ingredients of the powder component is partly or fully coated with a mixture of one or more dyes and a hydrophobic low-molecular or oligomeric organic bonding agent.

3. Dyed polymethylmethacrylate bone cement according to any one of the claims 1 or 2, **characterised in that** the dye or dyes are dissolved or suspended in the hydrophobic low-molecular or oligomeric organic bonding agent.

4. Dyed polymethylmethacrylate bone cement according to any one of the claims 1 - 3, **characterised in that** the mass ratio of dye/dyes and hydrophobic low-molecular or oligomeric organic bonding agent is 1.0 : 0.1 to 1.0 : 10.0.

5. Dyed polymethylmethacrylate bone cement according to any one of the claims 1 - 4, **characterised in that** oleic acid esters and/or elaidic acid esters and/or linoleic acid esters and/or linolenic acid esters of the aliphatic alcohols having 1 to 22 carbon atoms or oligomers of said esters are present as bonding agent.

6. Dyed polymethylmethacrylate bone cement according to any one of the claims 1 - 5, **characterised in that** methacrylic acid esters or acrylic acid esters of the aliphatic alcohols having 4 to 16 carbon atoms or oligomers of said methacrylic acid esters or acrylic acid esters having a molecular mass of less than 3,000 g/mol are present as bonding agent.

7. Dyed polymethylmethacrylate bone cement according to any one of the claims 1 - 6, **characterised in that** oleic acid, elaidic acid, linoleic acid, glycerol trioleate, glycerol elaidinate, glycerol trilinolenate, ethylene glycol dioleinate, ethylene glycol dielaidinate, ethylene glycol trilinolenate and oligomers thereof are present as bonding agent.

8. Dyed polymethylmethacrylate bone cement according to any one of the claims 1 - 7, **characterised in that** bonding agents are present that are produced by synthetic or partially synthetic means and do not contain proteins or degradation products of proteins.

9. Dyed polymethylmethacrylate bone cement according to any one of the claims 1 - 7, **characterised in that** refined peanut oil, hardened linseed oil, hardened rapeseed oil, and sunflower oil are present as bonding agent.

10. Dyed polymethylmethacrylate bone cement according to any one of the claims 1 - 9, **characterised in that** the bonding agent contains double bonds that can be polymerised by radical polymerisation.

11. Dyed polymethylmethacrylate bone cement according to any one of the claims 1 - 10, **characterised in that** mixtures of the bonding agent and the dye or dyes are soluble in methylmethacrylate.

12. Dyed polymethylmethacrylate bone cement according to any one of the claims 1 - 11, **characterised in that** chlorophyll, chlorophyllin, indigo, malachite green, crystal violet, brilliant blue, brilliant green, copper phthalocyanine, cobalt phthalocyanine, carotene, vitamin B12, and derivatives thereof are present as dyes.

13. Method for dyeing the polymer particles of the powder component of PMMA bone cements, **characterised in that** the polymer particles or mixtures of the polymer particles and X-ray contrast medium or mixtures of the polymer particles, X-ray contrast medium, and the initiator are coated with a liquid or pasty mixture of the dye or dyes and the bonding agent by mixing them in a temperature range from 0 °C to 50 °C in the presence of air or inert gas in a manner such that the thickness of the layer of the mixture that is formed on the coated particles is less than 2 µm and such that the coated particles do not stick to each other.

## Revendications

1. Ciment osseux de méthacrylate de polyméthyle coloré composé d'un composant liquide et d'un composant pulvérulent, **caractérisé en ce qu'**au moins la surface des particules polymères du composant pulvérulent est partiellement ou entièrement enduite d'un mélange d'un ou plusieurs colorants et d'un promoteur d'adhésion organique hydrophobe, à faible poids moléculaire ou oligomère, dans lequel une quantité de promoteur d'adhésion telle que les particules polymères ne sont pas gonflées de manière visuellement reconnaissable est présente.

2. Ciment osseux de méthacrylate de polyméthyle coloré selon la revendication 1, contenant en outre dans le composant pulvérulent du sulfate de baryum et/ou de l'oxyde de zirconium, **caractérisé en ce que** la surface de l'agent de contraste radiographique, sulfate de baryum et/ou oxyde de zirconium, ou la surface de tous les constituants du composant pulvérulent est aussi partiellement ou entièrement enduite d'un mélange d'un ou plusieurs colorants et d'un promoteur d'adhésion organique hydrophobe, à faible poids moléculaire ou oligomère.

3. Ciment osseux de méthacrylate de polyméthyle coloré selon l'une quelconque des revendications 1 et 2, **caractérisé en ce que** le ou les colorants sont dissous dans le promoteur d'adhésion organique hydrophobe, à faible poids moléculaire ou oligomère, ou en suspension dans celui-ci.

4. Ciment osseux de méthacrylate de polyméthyle coloré selon l'une quelconque des revendications 1 à 3, **caractérisé en ce que** le rapport massique entre colorant/colorants et promoteur d'adhésion organique hydrophobe, à faible poids moléculaire ou oligomère est de 1,0 sur 0,1 à 1,0 sur 10,0.

5. Ciment osseux de méthacrylate de polyméthyle coloré selon l'une quelconque des revendications 1 à 4, **caractérisé en ce que** des esters d'acide oléique et/ou des esters d'acide élaïdique et/ou des esters d'acide linoléique et/ou des esters d'acide linolénique des alcools aliphatiques avec 1 à 22 atomes de carbone ou des oligomères de ces esters sont présents en tant que promoteurs d'adhésion.

6. Ciment osseux de méthacrylate de polyméthyle coloré selon l'une quelconque des revendications 1 à 5, **caractérisé en ce que** des esters d'acide méthacrylique ou des esters d'acide acrylique des alcools aliphatiques avec 4 à 16 atomes de carbone ou des oligomères de ces esters d'acide méthacrylique ou esters d'acide acrylique avec une masse molaire inférieure à 3000 g/mole sont présents en tant que promoteurs d'adhésion.

7. Ciment osseux de méthacrylate de polyméthyle coloré selon l'une quelconque des revendications 1 à 6, **caractérisé en ce que** l'acide oléique, l'acide élaïdique, l'acide linolénique, le trioléate de glycérine, l'élaïdinate de glycérine, le trilinolénate de glycérine, le dioléinate d'éthylèneglycol, le diélaïdinate d'éthylèneglycol, le trilinolénate d'éthylèneglycol et leurs oligomères sont présents en tant que promoteurs d'adhésion.

8. Ciment osseux de méthacrylate de polyméthyle coloré selon l'une quelconque des revendications 1 à 7, **caractérisé en ce que** des promoteurs d'adhésion qui sont fabriqués par voie synthétique ou partiellement synthétique et qui ne contiennent aucune protéine ou aucun produit de dégradation de protéines sont présents.

9. Ciment osseux de méthacrylate de polyméthyle coloré selon l'une quelconque des revendications 1 à 7, **caractérisé en ce que** de l'huile d'arachide raffinée, de l'huile de lin durcie, de l'huile de colza durcie et de l'huile de tournesol sont présentes en tant que promoteurs d'adhésion.

10. Ciment osseux de méthacrylate de polyméthyle coloré selon l'une quelconque des revendications 1 à 9, **caractérisé en ce que** le promoteur d'adhésion contient des doubles liaisons polymérisables par voie radicalaire.

11. Ciment osseux de méthacrylate de polyméthyle coloré selon l'une quelconque des revendications 1 à 10, **caractérisé en ce que** des mélanges du promoteur d'adhésion et du colorant ou des colorants sont solubles dans du méthacrylate de méthyle.

12. Ciment osseux de méthacrylate de polyméthyle coloré selon l'une quelconque des revendications 1 à 11, **caractérisé en ce que** de la chlorophylle, de la chlorophylline, de l'indigo, du vert de malachite, du violet cristallin, du bleu brillant, du vert brillant, de la phtalocyanine de cuivre, de la phtalocyanine de cobalt, de la carotène, de la vitamine B12 et des dérivés qui en sont dérivés sont présents en tant que colorants.

13. Procédé de coloration des particules polymères du composant pulvérulent de ciment osseux de PMMA, **caractérisé en ce que** les particules polymères ou mélanges des particules polymères et de l'agent de contraste radiographique ou mélanges des particules polymères, de l'agent de contraste radiographique et de l'amorceur sont enduits avec un mélange liquide ou pâteux du colorant ou des colorants et du promoteur d'adhésion par mélange dans la plage de température de 0°C à 50°C en présence d'air ou de gaz inerte, de sorte que l'épaisseur de couche du mélange sur les particules enduites est inférieure à 2 µm et que les particules enduites ne sont pas collées ensemble.
